# EUROPEAN PATENT APPLICATION

(11) **EP 2 380 557 A1**
(43) Date of publication of application: **26.10.2011**
(21) Application number: 10731199.5
(22) Date of filing: 08.01.2010
(51) Int. Cl.: A61K 8/87, A61Q 1/00, A61Q 1/02, A61Q 1/12, A61Q 5/00, A61Q 15/00, A61Q 17/04, A61Q 19/00, A61Q 19/10, C11D 3/12, C11D 3/37, C11D 17/06, C11D 17/08

(54) **COSMETIC**

(30) Priority: 15.01.2009 JP 2009006875
(71) Applicant: Konishi Co., Ltd., Chuo-ku Osaka-shi Osaka 541-0045 (JP); Shiseido Co., Ltd., Tokyo 104-8010 (JP)
(72) Inventor: ORIGUCHI Toshiki, Osaka-shi Osaka 538-0053 (JP); NAKAYAMA Yoshitaka, Osaka-shi Osaka 538-0053 (JP); KURAHASHI Takuma, Yokohama-shi Kanagawa 224-8558 (JP); YAGI Katsuhiko, Yokohama-shi Kanagawa 224-8558 (JP); MURAKAMI Yuki, Yokohama-shi Kanagawa 224-8558 (JP)
(74) Representative: HOFFMANN EITLE
(86) International application number: PCT/JP2010/050124
(87) International publication number: WO 2010/082530

(57) **Abstract**

It is provided cosmetics to prevent to come off and keep long. The cosmetic comprises specific polyurethane particles with a cosmetic base. The specific polyurethane particle consists of a body of the polyurethane particle and hydrophilic fine silica powders existing on the body. The body is obtained by three-dimensionally polymerizing an isocyanate-terminated urethane prepolymer with trifunctional or more functional amines. The isocyanate-terminated urethane prepolymer is obtained by reacting polyisocyanates and polyols including a poly(tetramethylene ether)glycol. The polyurethane particles are obtained by spray-drying a mixed aqueous dispersion in which the hydrophilic fine silica powders and the polyurethane spheres are dispersed in a water.

## Description

### Technical Field

The present invention relates to contain polyurethane particles, especially, which superiorly absorb sebums.

### Background Art

It is used cosmetics consisting of polyurethane particles so as to smooth a skin or hide small wrinkles and pores. For example, the patent reference 1 discloses polyurethane particles which consist of a hexamethylene diisocyanate/trimethylol hexyllactone crosspolymer and silica.

Patent reference 1: JP2005-3114300 (the paragraph 0023 is referred.)

### Summary of Invention

### Technical Problem

The inventors have discovered that a specific polyurethane particle superiorly absorbs sebums (tokugan 2008-310341). Containing the polyurethane particles as one component of cosmetics, the inventors have assumed that the cosmetics give a smooth skin or hide small wrinkles and pores and keep long for superiorly absorbing sebums. So, experimenting with the cosmetics containing the polyurethane particles, the inventors have discovered that the cosmetics keep long. Therefore, the invention is a use invention for the cosmetics on the tokugan 2008-310341.

### Solution to Problem

The inventors is relates to the cosmetics containing polyurethane particles. Each surface of the bodies of the polyurethane particles is covered with hydrophilic fine silica powders. The body of the polyurethane particle is obtained by three-dimensionally polymerizing an isocyanate-terminated urethane prepolymer with trifunctional or more functional amines. And the isocyanate-terminated urethane prepolymer is obtained by reacting polyisocyanates and polyols including a poly(tetramethylene ether)glycol.

The polyurethane particle means one particle in the polyurethane particles. The polyurethane particle consists of the body of the polyurethane particle and the hydrophilic fine silica powders on the body.

The invention relates to the cosmetics containing polyurethane particles. The polyurethane particle is formed by covering the surface of the body with the hydrophilic fine silica powders.

The hydrophilic fine silica powder consists of mainly SiO₂, and has not substantially hydrophobic groups on the surface of it. The hydrophilic fine silica powders are well known, and sold by Nippon Aerosil Co. as AEROSIL 200, 200V, 200CF, 200FAD, 300, 300CF, 380, 50, 90G, 130, OX50, MOX80, MOX170, COK84 and etc.. Such hydrophilic fine silica powders are used in the invention. A particle size of the hydrophilic fine silica powder is finer than the particle size of the obtained polyurethane particle to cover the body of the polyurethane particle with the hydrophilic fine silica powders. Concretely, an average particle size of the obtained polyurethane particles is 1-50 µm, and an average particle size of the hydrophilic fine silica powders is 5-40 nm. Therefore, it is preferable that the particle size of the polyurethane particle is 20 or more times larger than the particle size of the hydrophilic fine silica powder. The average particle size of the hydrophilic fine silica powders is decided in nominal value.

It is used as the polyisocyanates for producing a polyurethane to be well known compounds which have two or more isocyanate groups in a molecule. It is preferably used alicyclic polyisocyanates or aliphatic polyisocyanates. Concretely, it is used an isophorone diisocyanate, a 1,6-hexamethylene diisocyanate and a hydrated MDI.

It is used as the polyols for producing a polyurethane to be a poly(tetramethylene ether)glycol. The other polyols may be mixed with the poly(tetramethylene ether)glycol. It may be used as the other polyols to be well known polyols for producing a polyurethane. A number average molecular weight of poly(tetramethylene ether)glycol is not limited, but preferably 650-3000.

Trifunctional or more functional amines for producing a polyurethane are used to three-dimensionally polymerize a urethane prepolymer obtained by reacting the polyols and the polyisocyanates. It is used a diethylenetriamine, triethylenetetramine, tetraethylenepentamine and 3,3'-diaminodipropylamine as the trifunctional or more functional amines.

The polyurethane particle of the invention is obtained by the following method with the above compound.

The method comprises; a process to obtain an isocyanate-terminated urethane prepolymer by reacting polyisocyanates and polyols including a poly(tetramethylene ether)glycol, a process to obtain an oil-in-water emulsion by adding and mixing the isocyanate-terminated urethane prepolymer to an aqueous solution in which a dispersant dissolves, a process to obtain polyurethane spheres in the oil-in-water emulsion by that the isocyanate-terminated urethane prepolymer in oil droplets is three-dimensionally polymerized by adding the trifunctional or more functional amines in the oil-in-water emulsion, a process to obtain an aqueous polyurethane dispersion in which the polyurethane spheres disperse by removing the dispersant from the oil-in-water emulsion. a process to obtain a mixed aqueous dispersion by mixing the aqueous polyurethane dispersion with an aqueous silica dispersion in which hydrophilic fine silica powders disperse in a water, and a process to spray-dry the mixed aqueous dispersion in a high temperature atmosphere with a spray-dryer. Thereby, the water is vaporized in the mixed aqueous dispersion, the polyurethane spheres are converted to bodies of the polyurethane particles, and each surface of the bodies of the polyurethane particles is covered with the hydrophilic fine silica powders. The polyurethane particles are obtained by the above method.

The processes of the method are more described as the followings. The isocyanate-terminated urethane prepolymer is obtained by reacting conventionally the polyisocyanates and the polyols including a poly(tetramethylene ether)glycol. Then, an amount by mole of NCO group of the polyisocyanates exceeds an amount by mole of OH group of the polyols to terminate at the NCO group in the urethane prepolymer. The amount by mole of NCO group is conventionally 1.5-3.0 times to the amount by mole of OH group. Generally, the polyisocyanates and polyols are dissolved in an organic solvent such as ethyl acetate or methyl ethyl ketone, and react with a tin catalyst such as dibutyl tin dilaurate or an amine catalyst such as 1,8-diazabicyclo[5.4.0]undec-7-ene.

Conventionally, it is used only the poly(tetramethylene ether)glycol as the polyols, but may be mixed with the other polyols. It may be used a polyalcohol, polyetherpolyol, polyesterpolyol, polycarbonatepolyol, polyolefinpolyol, polyacrylicpolyol or castor oil as the other polyols. Mixing with the other polyols, it is preferable that the poly(tetramethylene ether)glycol occupies 50 or more mass% in the polyols. Less than 50 mass%, the polyurethane particle is insufficient to absorb sebums because the paly(tetramethylene ether)glycol is a component to absorb sebums. Using only the poly(tetramethylene ether)glycol, the body of the polyurethane particle tends to be soft. Mixing with the polycarbonatepolyol as the other polyols, the body of the polyurethane particle tends to be gradually hard with increase of the content.

The content of the isocyanate group (NCO group) of the isocyanate-terminated urethane prepolymer is 2.0-10 mass%, and preferably 2.5-3.5 mass%. More than 10 mass% in the content, the polyurethane particle is insufficient to absorb sebums because a content of urethane linkage is much. Less than 2.0 mass% in the content, it may be difficult to treat the isocyanate-terminated urethane prepolymer because it becomes high molecular weight and high viscosity.

The obtained isocyanate-terminated urethane prepolymer is added and mixed to an aqueous solution in which a dispersant is dissolved to obtain an oil-in-water emulsion. Concretely, the oil-in-water emulsion is obtained by the following process.

It is prepared the aqueous solution in which the dispersant is dissolved. The dispersant is to emulsify the isocyanate-terminated urethane prepolymer in a water. For example, as the dispersant, it is used general surfactants such as nonionic surfactant or usual polymeric dispersants such as polyvinyl alcohol. The dispersant is dissolved in the water to obtain the aqueous solution. Dissolving the dispersant, it is preferable to heat the water so as to dissolve easily. After the dispersant is entirely dissolved in the water, it is preferable to cool the aqueous solution at room temperature. A temperature of the heated water is decided at a solubility of the dispersant. For example, using the polyvinyl alcohol having a saponification degree of about 86.5-89.0 mol%, the temperature of the heated water is about 90°C. A concentration of the dispersant is decided so as to emulsify the isocyanate-terminated urethane prepolymer in the water. It is conventionally about 3-20 mass%.

The isocyanate-terminated urethane prepolymer is added and mixed in the aqueous solution cooled at room temperature. An addition mass of the isocyanate-terminated urethane prepolymer is conventionally about 3-20 parts by mass to 100 parts by mass of the aqueous solution. The machine and degree for mixing are decided so as to emulsify the isocyanate-terminated urethane prepolymer in the water. It is used a homomixer, a homogenizer, a high-pressure homogenizer or an ultrasonic homogenizer as the machine for mixing. Using the homomixer, the degree for mixing is about 5 minutes at about 8000 rpm. Adding and mixing are conventionally executed at room temperature, but may be executed at a little heating temperature so as to mix easily.

A trifunctional or more functional amine is added in the oil-in-water emulsion. The trifunctional or more functional amine is used to obtain polyurethane having network structure by three-dimensionally polymerizing the isocyanate-terminated urethane prepolymer as described in paragraph 0011. It is used various poly-functional amine as the trifunctional or more functional amine. The poly-functional amine is added in itself or in solution dissolving it in a solvent such as water. An addition mass of the poly-functional amine is preferably chemical equivalent to the isocyanate group of the isocyanate-terminated urethane prepolymer in the oil-in-water emulsion. That is, the poly-functional amine is preferably added such as an amino group of the poly-functional amine is Chemical equivalent to the isocyanate group of the isocyanate-terminated urethane prepolymer. Thereby, the isocyanate group reacts with the amino group to three-dimensionally polymerize. The addition mass of the poly-functional amine may be from 0.8 to less than the chemical equivalent to restraint three-dimensional polymerization. Furthermore, the addition mass of the poly-functional amine may be to 1.2 from more than the chemical equivalent to be densely three-dimensional polymerization.

Not only the poly-functional amine such as the trifunctional or more functional amine but also a difunctional amine or polyalcohol may be added with the poly-functional amine to react with isocyanate group. Using the difunctional amine or polyalcohol, it may be controlled to three-dimensionally polymerize the isocyanate-terminated urethane prepolymer.

Three-dimensionally polymerizing the isocyanate-terminated urethane prepolymer in oil droplets with the addition of the poly-functional amine, the oil-in-water emulsion is conventionally heated to quicken the reaction of the polymerization. The temperature of the oil-in-water emulsion is decided on the reactivity of the poly-functional amine. For example, using the 3,3'-diaminodipropylamine as the poly-functional amine, the oil-in-water emulsion is heated at about 80 °C, then the three-dimensional polymerization has finished for about 20 hours.

After the three-dimensional polymerization is finished, the oil droplets in the oil-in-water emulsion are converted to the polyurethane spheres. That is, the polyurethane spheres are dispersed to be in a dispersion. The dispersant in the dispersion is removed. The method of removing the dispersant is the following. The polyurethane spheres are collected with filtering or centrifugal separating the dispersion. The collected polyurethane spheres are dispersed in a water again. The polyurethane spheres are again collected with filtering or centrifugal separating the second dispersion. By repeating the above process, it is obtained an aqueous polyurethane dispersion in which the polyurethane spheres disperse as dispersion medium of water.

It is prepared the aqueous silica dispersion in which the hydrophilic fine silica powders disperse into a water. It is easily prepared to add and mix the hydrophilic fine silica powders in the water. It is decided the condition of adding and mixing or the content of the powders so as to disperse the powders informally in the water, not cohering. For example, using the trade mark ┌Aerosil® 200┘ which are the hydrophilic fine silica powders and dispersing them in a content of 5 mass%, the aqueous silica dispersion is easily obtained by mixing with a water for 10 minutes at 10000 rpm by a homomixer.

Mixing the above aqueous silica dispersion with the above aqueous polyurethane dispersion, the mixed aqueous dispersion is obtained. The mixing ratio of the both is decided on the ratio of the solid content of the both. Mixing the both, it is preferable to add a water so as to prevent that the hydrophilic fine silica powders and polyurethane spheres cohere. The addition mass of the water is optionally decided, conventionally being about equal to the mass of the aqueous polyurethane dispersion. Adding the water, it is preferable to mix so as to prevent coherence.

The mixed aqueous dispersion is dried with the spray-dryer having nozzles or disks. Using the spray-dryer having nozzles, the mixed aqueous dispersion is spray-dried through each nozzle in the high temperature atmosphere to dry. Using the spray-dryer having disks, the mixed aqueous dispersion is dropped on each rotating disk and spray-dried on the centrifugal force of each disk in the high temperature atmosphere to dry. The spray-drying in the invention is characterized by spraying in remaining the form of each polyurethane sphere in the mixed aqueous dispersion, that is, not atomizing the polyurethane sphere. Because the polyurethane sphere is three-dimensionally polymerized in the mixed aqueous dispersion. Spraying the mixed aqueous dispersion, it is sprayed the polyurethane spheres and the hydrophilic fine silica powders with the water. Drying the water, the hydrophilic fine silica powders adhere onto each surface of the polyurethane spheres. The polyurethane spheres being softer, the hydrophilic fine silica powders adhere on the surface in implanting.

The condition of spraying is not strict because the polyurethane spheres are not atomized at spraying. It is not strict the condition of the pressure, centrifugal force and aperture of the nozzle for atomization. Therefore, the spray-dryer having nozzles or disks may be used. The condition of the high temperature atmosphere to dry after spraying is decided so as to vapor the spraying water. The condition is conventional, for example, the inlet of a hot air may be the temperature of about 140°C and the temperature of about 70°C into the dryer. Spraying and drying the polyurethane spheres, each body of the polyurethane particles is formed with one of them or joining two or more. The polyurethane sphere being softer or more stick surface, the body of the polyurethane particles tends to be formed with two or more of the polyurethane spheres. The polyurethane spheres being harder, the body of the polyurethane particles tends to be formed with one of the polyurethane spheres.

Spray-drying the mixed aqueous dispersion, the polyurethane sphere is converted to the body of the polyurethane particle, covering the surface of the body with the hydrophilic fine silica powders. The hydrophilic fine silica powders adhere on the body. Being softer the polyurethane sphere or the body of the polyurethane particle, the hydrophilic fine silica powders adhere on the body in implanting. An average particle size of the polyurethane particles is decided on an average particle size of the polyurethane sphere in the mixed aqueous dispersion, a softness or stickiness of the body or the condition of spray-drying. The average particle size of the polyurethane particles is conventionally about 1-50 µm. The average particle size is measured with the following method. 100 polyurethane particles are picked up, and each particle size is measured with an optical microscope or an electron microscope. The average particle size is calculated on a number average. The picked up polyurethane particle cohering with two or more, the cohered particle is separated one by one and measured.

The polyurethane particles may be compounded with a cosmetic base as it is or after applying a hydrophobic treatment such as siliconization or fluoridization to them. The polyurethane particle superiorly absorbs the sebums and oleic acids, but partly absorbs other oils. Therefore, it may be permitted to absorb and hold various oils, medicinal properties, ultraviolet absorbers, moisturizing agents, emollient agents, perfumed oils, germicidal agents or antioxidants in the polyurethane particles so as not to lower an absorbent capacity of sebums. The containing mass of the polyurethane particles is optionally decided, conventionally 0.1-30 parts by mass, preferably 1-20 parts by mass, more preferably 3-15 parts by mass to the amount of the cosmetic.

It is used a general base as the cosmetic base in the invention. It is preferably used the cosmetic base which is difficult to absorb oils because the polyurethane particles partly absorb oils. The form of the cosmetic is optionally decided, generally is selected from the group consisting of solid, powder, gel and liquid. A solid cosmetic has somewhat volume by molding the cosmetic base. The solid cosmetic is used as a face-wash or foundation. A powder cosmetic is observed as powders, and the containing mass of the powders is 80 or more parts by mass to the amount of the powder cosmetic. The powder cosmetic is used as a foundation, face powder, eye shadow, rouge, body powder, deodorant powder or fragrance powder. A gel cosmetic is used as a hairdressing, foundation materials or hand cream. A liquid cosmetic is observed as an aqueous solution or emulsion. The liquid cosmetic is used as a toner, milky lotion, foundation materials or hairdressing. It may be used as a sheet-like cosmetic which contains the solid cosmetic or the liquid cosmetic in a sheet such as non-woven fabric. Wiping a skin with the sheet-like cosmetic, the solid cosmetic or the liquid cosmetic is applied to the skin.

The cosmetics may be used with the polyurethane particles absorbing a well known active ingredient. For example, the cosmetics may be used as a make-up preparations, skin care preparations, pimple care preparations, antiperspirants, ultraviolet protective preparations, hair cosmetics, face packs or fragrance cosmetics. The cosmetics keep long because the active ingredient is gradually released from the polyurethane particles.

### Advantageou Effects of invention

The cosmetics contain the polyurethane particles which superiorly absorb the sebums. Applying the cosmetics to a skin or hair, the secreted sebums on the skin or hair are superiorly absorbed. Therefore, a makeup is prevented to come off because the sebums are difficult to pile up between the cosmetics and the skin or hair. That is, applying the cosmetics of the invention, the makeup is prevented to come off and keep long.

### Examples

The invention is described on the basis of the following examples, but the invention is not limited to the example. The invention must be constructed on the discovery that the specific polyurethane particle superiorly absorbs the sebums.

### Producing example 1 for polyurethane particles 1

### [Preparing an isocyanate-terminated urethane prepolymer]

20 parts by mass of ethyl acetate and 300 parts by mass of poly(tetramethylene ether)glycol having a number average molecular weight of 1000 were charged in a four-necked flask fitted with a stirrer, nitrogen inlet, thermometer and condenser. 104.6 parts by mass of isophorone diisocyanate and 0.04 parts by mass of dibutyl tin dilaurate were added and reacted under a nitrogen stream at 75-80 °C for 5 hours to obtain the isocyanate-terminated urethane prepolymer containing 3.6% of isocyanate group.

### [Preparing an oil-in-water emulsion]

900 parts by mass of deionized water and 100 parts by mass of polyvinyl alcohol having a saponification degree of 86.5-89.0 mol% as a dispersant which was sold as a trade name of PVA-205 by Kuraray Co., Ltd. were charged and heated at 90°C to obtain an aqueous solution of polyvinyl alcohol. 100 parts by mass of the above prepared isocyanate-terminated urethane prepolymer were added in the aqueous solution of polyvinyl alcohol, and they were mixed at 8000 rpm for 5 minutes with a homomixer to obtain the oil-in-water emulsion in which oils are consisted of the isocyanate-terminated urethane prepolymer.

### [Preparing an aqueous polyurethane dispersion]

35.7 parts by mass of 10% aqueous solution of 3,3'-diaminodipropylamine were added in the above oil-in-water emulsion. They were heated at 80 °C with stirring, remained at the temperature and reacted for 20 hours. The isocyanate-terminated urethane prepolymer reacted with the 3,3'-diaminodipropylamine to be three-dimensionally polymerized, and polyurethane spheres were formed. The polyurethane spheres were sunk and collected with a centrifugation. The polyurethane spheres were again dispersed in a water and were sunk and collected with a centrifugation. Sixth dispersing and collecting were carried out to remove the polyvinyl alcohol as the dispersant from the water. It is obtained the aqueous polyurethane dispersion comprising the polyurethane spheres of 40% by mass.

### [Preparing an aqueous silica dispersion]

95 parts by mass of deionized water were charged and stirred at 10000 rpm in a homomixer. 5 parts by mass of hydrophilic fine silica powders having an average particle size of 20 nm which was sold as a trade name of AEROSIL 200 by Nippon Aerosil Co., Ltd. were added in the deionized water and mixed for 10 minutes to obtain the aqueous silica dispersion.

### [Preparing a mixed aqueous dispersion]

100 parts by mass of the above aqueous polyurethane dispersion, 80 parts by mass of the above aqueous silica dispersion and 110 parts by mass of deionized water were mixed to obtain the mixed aqueous dispersion.

### [Producing polyurethane particles 1]

Spray-drying the above mixed aqueous dispersion with a spray-dryer which was sold as a trade name of L-81 by Ohkawara Kakohki Co., Ltd., the polyurethane particles were obtained. Each condition of the spray-drying was as the following. A pressure of spraying was 0.3MPa, a temperature of an inlet of a hot air was 140 °C and a temperature into a dryer was 70 °C. Vaporizing the water in the mixed aqueous dispersion by the spray-drying, the polyurethane spheres were converted to bodies of the polyurethane particles and the hydrophilic fine silica powders were adhered to each surface of the bodies of the polyurethane particles in implanted. Therefore, each body of the polyurethane particles was covered with the hydrophilic fine silica powders to be the polyurethane particle. The polyurethane particles were not cohered and smooth because of separating from each one. An average particle size of the polyurethane particles is about 12µm.

### Producing example 2 for polyurethane particles 2

### [Preparing an isocyanate-terminated urethane prepolymer]

The isocyanate-terminated urethane prepolymer containing 2.5% of isocyanate group was obtained with the same method of the Producing example 1 except that a poly(tetramethylene ether)glycol having a number average molecular weight of 3000 was used in place of the number average molecular weight of 1000 and 45.0 parts by mass of isophorone diisocyanate were used in place of the 104.6 parts by mass.

### [Preparing an oil-in-water emulsion]

The oil-in-water emulsion was obtained with the same method of the Producing example 1 except that the above isocyanate-terminated urethane prepolymer was used.

### [Preparing an aqueous polyurethane dispersion]

The aqueous polyurethane dispersion was obtained with the same method of the Producing example 1 except that the above oil-in-water emulsion was used and 24.5 parts by mass of 10% aqueous solution of 3,3'-diaminodipropylamine were used in place of the 35.7 parts by mass.

### [Preparing an aqueous silica dispersion]

The aqueous silica dispersion was obtained with the same method of the Producing example 1.

### [Preparing a mixed aqueous dispersion]

The mixed aqueous dispersion was obtained with the same method of the Producing example 1 except that the above aqueous polyurethane dispersion was used.

### [Producing polyurethane particles 2]

The polyurethane particles were obtained with the same method of the Producing example 1 except that the above mixed aqueous dispersion was used. The properties of the polyurethane particles were the same as them of the polyurethane particles 1. An average particle size of the polyurethane particles is about 15 µ m.

### Example 1

It was obtained a powder cosmetic consisting of the following composition. The powder cosmetic was used as a foundation.

| | |
|---|---|
| the polyurethane particles 1 | 5 masts% |
| mica treated by silicon | 20 mass% |
| mica | 5mass% |
| titanium oxide coated with polymethylhydrosiloxane | 10 mass% |
| fine particle of titanium oxide | 5 mass% |
| iron oxide treated by alkyl | 3.44 mass% |
| silicone elastomer as trade name KSP-100 by Shin-Etsu Chemical Co., Ltd. | 8 mass% |
| activated zinc oxide | 8.5 mass% |
| mica with barium sulfate and aluminum oxide | 6 mass% |
| chlorphenesin | 0.2 mass% |
| olefin oligomer | 1 mass% |
| poly(methylphenylsiloxane) | 2 mass% |
| diisostearyl malate | 2.5 mass% |
| 2-ethylhexyl paramethoxycinnamate | 3 mass% |
| sorbitan sesquiisostearate | 1.2 mass% |
| tocopherol | 0.02 mass% |
| talc coated with poly(methylhydrosiloxane) | residue |

### Example 2

A powder cosmetic was obtained with the same method of the Example 1 except that the blending quantities of the polyurethane particles 1 and the silicone elastomer were changed as the followings.

| | |
|---|---|
| the polyurethane particles 1 | 10 mass% |
| silicone elastomer as trade name KSP-100 by Shin-Etsu Chemical Co., Ltd. | 3 mass% |

### Example 3

A powder cosmetic was obtained with the same method of the Example 1 except that the blending quantity of the polyurethane particles 1 was changed as the following, and the silicone elastomer was not used.

| | |
|---|---|
| the polyurethane particles 1 | 13 mass% |
| silicone elastomer as trade name KSP-100 by Shin-Etsu Chemical Co., Ltd. | not used |

### Comparative Example 1

A powder cosmetic was obtained with the same method of the Example 1 except that the polyurethane particles 1 were not used, and the blending quantity of the silicone elastomer was changed as the following.

| | |
|---|---|
| the polyurethane particles 1 | not used |
| silicone elastomer as trade name KSP-100 by Shin-Etsu Chemical Co., Ltd. | 13 mass% |

### Comparative Example 2

A powder cosmetic was obtained with the same method of the Example 1 except that polyurethane particles sold as trade name PLASTIC POWDES D-400 by Toshiki Pigment Co., Ltd. were used in place of the polyurethane particles 1. The polyurethane particles (PLASTIC POWDES D-400) consist of a hexamethylene diisocyanate/trimethylol hexyllactone crosspolymer and silica, and an average particle size of them is about 13 µm.

20 experts used each powder cosmetic of the example 1-3, the comparative example 1 and 2. They evaluated a keeping after 2 hours, a spreading in putting make-up, a smoothness and a hiding small wrinkles and pores on the basis of the following scores.
a score of 5: very superior
a score of 4: superior
a score of 3: medium
a score of 2: inferior
a score of 1: very inferior

Each average score was calculated. A practical evaluation was decided on the basis of the following. The result was shown in the table 1.
⊚: more than 4.4 of the average score.
○: 3.5-4.4 of the average score
△: 2.6-3.5 of the average score
×: less than 2.6 of the average score

**[Table 1]**

| | Keeping | Spreading | Smoothness | Hiding |
|---|---|---|---|---|
| Example 1 | ○ | ○ | ○ | ○ |
| Example 2 | ⊚ | ⊚ | ⊚ | ⊚ |
| Example 3 | ⊚ | ⊚ | ⊚ | ⊚ |
| Compar.Ex.1 | △ | ○ | ○ | ○ |
| Compar.Ex.2 | △ | △ | △ | △ |

The table 1 shows that the powder cosmetics of the example 1-3 using the polyurethane particles 1 is superior in the keeping to the comparative example 1 not using the polyurethane particles 1 and the comparative example 2 using the other sold polyurethane particles. That shows to prevent that a makeup comes off because the polyurethane particles 1 much absorb sebums.

### Example 4

It was obtained a powder cosmetic consisting of the following composition. The powder cosmetic was used as a loose powder.

| | |
|---|---|
| the polyurethane particles 2 | 8.5 mass% |
| mica treated by silicon | 20 mass% |
| iron oxide | 0.05 mass% |
| activated zinc oxide | 1 mass% |
| zinc myristate | 4 mass% |
| talc coated with poly(methylhydrosiloxane) | residue |

### Comparative Example 3

A powder cosmetic was obtained with the same method of the Example 4 except that polyurethane particles sold as trade name PLASTIC POWDES D-400 by Toshiki Pigment Co., Ltd. were used in place of the polyurethane particles 2. The polyurethane particles (PLASTIC POWDES D-400) consist of a hexamethylene diisocyanate/trimethylol hexyllactone crosspolymer and silica, and an average particle size of them is about 13 µm.

### Comparative Example 4

A powder cosmetic was obtained with the same method of the Example 4 except that the silicone elastomer sold as trade name KSP-100 by Shin-Etsu Chemical Co., Ltd. was used in place of the polyurethane particles 2.

Each powder cosmetic of the example 4, the comparative example 3 and 4 was evaluated on the same, and shown in the table 2.

**[Table 2]**

| | Keeping | Spreading | Smoothness | Hiding |
|---|---|---|---|---|
| Example 4 | ⊚ | ○ | ⊚ | ○ |
| Compar.Ex.3 | △ | ○ | △ | ○ |
| Compar.Ex.4 | △ | ○ | ○ | ○ |

The table 2 shows that the powder cosmetic of the example 4 using the polyurethane particles 2 is superior in the keeping to the comparative example 3 using the other sold polyurethane particles and the comparative example 4 using the sold silicone elastomer. That shows to prevent that a makeup comes off because the polyurethane particles 2 much absorb sebums.

### Example 5

It was obtained a powder cosmetic consisting of the following composition. The powder cosmetic was used as a powdery foundation.

| | |
|---|---|
| the polyurethane particles 1 | 5 mass% |
| polydimethylsiloxane | 5 mass% |
| isostearic acid | 0.5 mass% |
| diisostearyl malate | 3 mass% |
| triethylhexanoin | 1 mass% |
| sorbitan sesquiisostearate | 1 mass% |
| mica coated with spherical PMMA (polymethyl methacrylate) | 6 mass% |
| pearl agent | 1 mass% |
| fine particle of zinc oxide | 0.5 mass% |
| fine particle of titanium oxide | 2 mass% |
| synthetic fluotphlogopite | 2 mass% |
| talc treated by soap scum | 8 mass% |
| vitamin E acetate | 0.1 mass% |
| δ -tocopherol | 0.1 mass% |
| ethylparaben | appropriate mass% |
| trimethoxycinnamic acid methylbis(trimethylsiloxy)silylisopentyl | 1 mass% |
| 2-ethylhexyl p-methoxycinnamate | 1 mass% |
| spherical powders of polyalkyl acrylate | 6 mass% |
| sericite coated with poly(methylhydrosiloxane) | 20 mass% |
| titanium oxide coated with poly(methylhydrosiloxane) | 15 mass% |
| pigment coated with poly(methylhydrosiloxane) [colorant] | 5 mass% |
| talc coated with poly(methylhydrosiloxane) | residue |

### Example 6

It was obtained a powder cosmetic consisting off the following composition. The powder cosmetic was used as a powdery foundation.

| | |
|---|---|
| the polyurethane particles 1 | 8 mass% |
| particles of synthetic hydrocarbon wax | 2 mass% |
| polydimethylsiloxane | 6 mass% |
| purified lanolin | 5 mass% |
| triethylhexanoin | 2 mass% |
| sorbitan sesquiisostearate | 0.5 mass% |
| needle-like particles of titanium oxide | 5 mass% |
| fine particles of zinc oxide | 1 mass% |
| particles sinterring iron oxide and titanium oxide coated with silicone | 7 mass% |
| barium sulfate | 8 mass% |
| mica deoxidized by titanium [titanium pearl pigment] | 2 mass% |
| synthetic fluorphlogopite coated with silicone | 5 mass% |
| talc coated with silicone | 2 mass% |
| mica coated with silicone | 15 mass% |
| stearyl glycyrrhetinate | 0.1 mass% |
| ascorbyl dipalmitate | 0.1 mass% |
| D- α-tocopherol acetate | 0.1 mass% |
| Do- δ -tocopherol | 0.1 mass% |
| paraoxy benzoic acid ester | appropriate mass% |
| 2-ethylhexyl paramethoxycinnamate | 3 mass% |
| bengara coated with silicone | 1 mass% |
| yellow iron oxide coated with silicone | 1 mass% |
| black iron oxide | 1mass% |
| spherical powders of polyalkyl acrylate | 3 mass% |
| perfume | appropriate mass% |
| calcined sericite | residue |

### Example 7

It was obtained a liquid, cosmetic consisting of the following composition. The liquid cosmetic was used as a toner.

| | |
|---|---|
| the polyurethane particles 1 | 3 mass% |
| ethanol | 5 mass% |
| glycerol | 1 mass% |
| 1,3-butanediol | 5 mass% |
| polyoxyethylene polyoxypropylene decyltetradecyl ether | 0.2 mass% |
| sodium hexametaphosphate | 0.03 mass% |
| trimethylglycine | 1 mass% |
| poly(sodium aspartate) | 0.1 mass% |
| α -tocopherol-2-L-ascorbate diesterpotassium phosphate | 0.1 mass% |
| thiotaurine | 0.1 mass% |
| green tea extract | 0.1 mass% |
| peppermint extract | 0.1 mass% |
| iris germanica florentina extract | 0.1 mass% |
| EDTA3Na | 0.1 mass% |
| carboxyvinyl polymer | 0.05 mass% |
| potassium | 0.02 mass% |
| phenoxyethanol | appropriate mass% |
| perfume | appropriate mass% |
| purified water | residue |

### Example 8

It was obtained a liquid cosmetic consisting of the following composition. The liquid cosmetic was used as a toner.

| | |
|---|---|
| the polyurethane particles 1. | mass% |
| ethanol | 10 mass% |
| glycerol | 2 mass% |
| poly(oxyethylene · oxypropylene) · methylpolysiloxane copolymer | 1 mass% |
| lauryl dimethyl aminoacetic acid betaine | 0.1 mass% |
| citric acid | 0.02 mass% |
| sodium citrate | 0.08 mass% |
| sodium hexametaphosphate | 0.01 mass% |
| hypotaurine | 0.1 mass% |
| chamomile extract | 0.1 mass% |
| lavender oil | 0.001 mass% |
| phenoxyethanol | appropriate mass% |
| active hydrogen water | 1 mass% |
| purified water | residue |

### Example 9

It was obtained a liquid cosmetic consisting of the following composition. The liquid cosmetic was used as a toner.

| | |
|---|---|
| the polyurethane particles 1 | mass% |
| ethanol | 40 mass% |
| dipropylenglycol | 1 mass% |
| polyoxyethylene polyoxypropylene decyltetradecyl ether | 0.1 mass% |
| anhydrous silicic acid | 1 mass% |
| salicylic acid | 0.1 mass% |
| sodium citrate | 0.2 mass% |
| zinc p-phenolsulfonate | 0.2 mass% |
| dipotassium glycyrrhizinate | 0.1 mass% |
| pyridoxine hydrochloride | 0.1 mass% |
| 1-menthol | 0.05 mass% |
| EDTA3Na | 0.05 mass% |
| bentonite | 0.8 mass% |
| purified water | residue |

### Example 10

It was obtained a powder cosmetic consisting of the following composition. The powder cosmetic was used as a deodorant spray.

| | |
|---|---|
| the polyurethane particles 1 | 1.5 mass% |
| aluminum potassium sulfate (an average particle was 20 µm.) | 3 mass% |
| zeolite supporting silver ion, zinc ion and ammonium ion (an average particle was bout 5 µm, and the particles more than 15 µm of a particle size were less 1 mass%.) | 2 mass% |
| zinc oxide | 0.2 mass% |
| (oil components) | |
| polv(oxyethylene)nonylphenylether | 0.5 mass% |
| dimetnylpolysiloxane (20 mPa - s, 25 °C) | 0.1 mass% |
| isopropyl myristate | 0.5 mass% |
| (addition agents) | |
| polyoxyethylene sorbitan monooleate | 0.1 mass% |
| perfume | 0.1 mass% |
| (propellant) | |
| liquefied petroleum gas | 92 mass% |

The deodorant spray was obtained by the following method. Powder components were mixed with a kneader. The oil components were mixed with a blender. The addition agents were absorbed into the mixed oil components. The mixed powder components and the mixed oil components absorbing the addition agents each were charged in a spray can, and the propellant was charged in the spray can to obtain the deodorant spray.

### Example 11

It was obtained a powder cosmetic consisting of the following composition. The powder cosmetic was used as a loose powder.

| | |
|---|---|
| the polyurethane particles 1 | 20 mass% |
| titanated mica coated with an alkyl modified silicone resin | 25 mass% |
| sericite coated with an alkyl modified silicone resin | 30 mass% |
| sapindus mukurossi peel extract | 0.01 mass% |
| hemp cellulose powders | 0.1 mass% |
| talc coated with an alkyl modified silicone resin | residue |

### Example 12

It was obtained a liquid cosmetic consisting of the following composition. The liquid cosmetic was used as a makeup base of water-in-oil emulsion.

| | |
|---|---|
| the polyurethane particles 1 | 3 mass% |
| dimethylpolysiloxane (6 mPa · s) | 5 mass% |
| decamethylcyclopentasiloxane | 30 mass% |
| polyoxyethylene · methylpolysiloxane copolymer | 3 mass% |
| dodecamethylcyclohexasiloxane | 1 mass% |
| glycerol | 5 mass% |
| dipropylglycol | 5 mass% |
| sage oil | 0.1 mass% |
| talc | 0.1 mass% |
| titanated mica | 0.1 mass% |
| polymethylsilsesquioxane powders | 10 mass% |
| tocopherol acetate | 0.1 mass% |
| δ - tocopherol | 0.1 mass% |
| thiotaurine | 0.1 mass% |
| peppermint extract | 0.1 mass% |
| p-oxybenzoic acid ester | appropriate mass% |
| phenoxyethanol | appropriate mass% |
| edetate trisodium | appropriate mass% |
| colored pigment | appropriate mass% |
| dimethyl distearyl ammonium hectorite | 1.5 mass% |
| purified water | residue |

### Example 13

It was obtained a liquid cosmetic consisting of the following composition. The liquid cosmetic was used as a milky foundation.

| | |
|---|---|
| the polyurethane particles 1 | 4 mass% |
| microcrystalline wax | 1 mass% |
| dimethylpolysiloxane | 15 mass% |
| decamethylcyclohexasiloxane | 2 mass% |
| 1,3-butanediol | 6 mass% |
| candelilla wax | 3 mass% |
| isostearic acid | 1 mass% |
| ethylene glycol fatty acid ester | 0.1 mass% |
| octyldodecyl lanolate | 0.5 mass% |
| 2-alkyl-N-carboxymethyl-N-hydroxyethyl imidazolinium betaine | 4 mass% |
| titanium oxide | 14.5 mass% |
| barium sulfate | 5 mass% |
| talc | 3 mass% |
| (dim.ethicone/vinyldimethicone)crosspolymer | powders 0.1 mass% |
| sodium metaphosphate | 0.1 mass% |
| hydroxypropyl- β -cyclodextrin | 0.1 mass% |
| DL- α'tocopherol acetate | 0.1 mass% |
| hamamelis virginiana (witch hazel) extract liquid | 0.1 mass% |
| peony root extract | 0.1 mass% |
| sodium chondroitin sulfate | 0.1 mass% |
| sodium hyaluronate | 0.1 mass% |
| p-oxybenzoic acid ester | appropriate mass% |
| bengara | appropriate mass% |
| yellow iron oxide | appropriate mass% |
| black iron oxide | appropriate mass% |
| xanthan gum | 0.2 mass% |
| carboxymethyl cellulose | 0.2 mass% |
| melilot extract | 2 mass% |
| purified water | residue |

### Example 14

It was obtained a gel cosmetic consisting of the following composition. The gel cosmetic was used as a hair styling wax.

| | |
|---|---|
| the polyurethane particles 1 | 10 mass% |
| microcrystalline wax | 5 mass% |
| ceresin | 10 mass% |
| sunflower oil | 1 mass% |
| diglycerolsorbitan pentaoctanoate | 10 mass% |
| lipophilic glyceryl monostearate | 5 mass% |
| self-emulsified glyceryl monostearate | 5 mass% |
| silanized silicic anhydride | 5 mass% |
| pentaerythritol Tetra-2-ethylhexanoate | residue |

### Example 15

It was obtained a powder cosmetic consisting of the following composition. The powder cosmetic was used as a powder-type body soap.

| | |
|---|---|
| the polyurethane particles 1 | 3 mass% |
| sodium cocoyl ethyl ester sulfonate | 10 mass% |
| sodium methyl lauroyl taurate | 1 mass% |
| potassium N-myristoyl-L-glutamate | 10 mass% |
| sodium N-lauroyl-L-glutamate | 20 mass% |
| POLYMER JR-400 sold by Union Carbide Co. | 0.2 mass% |
| talc | 10 mass% |
| hydroxypropyl methyl cellulose | 0.2 mass% |
| dipotassium glycyrrhiznate | 0.1 mass% |
| trimethylglycine | 0.1 mass% |
| sophora flavescens extract | appropriate mass% |
| saxifraga sarmentosa extract | appropriate mass% |
| perilla extract | appropriate mass% |
| D-mannitol | residue |

### Example 16

It was obtained a gel cosmetic consisting of the following composition. The gel cosmetic was used as a gel-type makeup base.

| | |
|---|---|
| the polyurethane particles 1 | 7 mass% |
| ethanol | 4 mass% |
| 1,3-butanediol | 7 mass% |
| glycerol | 8 mass% |
| hydrophobic modifies polyetherurethane | 2 mass% |
| succinoglucan | 0.15 mass% |
| citric acid | 0.02 mass% |
| sodium citrate | 0.08mass% |
| iron oxide | 0.01 mass% |
| phenoxyethanol | 0.5 mass% |
| edetate trisodium | 0.1 mass% |
| purified water | residue |

### Example 17

It was obtained a liquid cosmetic consisting of the following composition. The liquid cosmetic was used as a sunscreen preparation.

| | |
|---|---|
| the polyurethane particles 1 | 5 mass% |
| decamethylcyclopentasiloxane | 20 mass% |
| trimethylsiloxysilicic acid | 1 mass% |
| polyoxyethylene methylpolysiloxane copolymer | 2 mass% |
| dipropylglycol | 4 mass% |
| squalane | 5 mass% |
| fine particles titanium oxide coated with silicone (an average size was 20 nm) | 10 mass% |
| talc (hydrophobic treatment) | 6 mass% |
| paraben | appropriate mass% |
| phenoxyethanol | appropriate mass% |
| edetate trisodium | 0.02 mass% |
| 4-t-butyl-4'-methoxydibenzoylmethane | 0.1 mass% |
| 2-ethylhexyl p-methoxycinnamate | 7 mass% |
| diparamethoxycinnamic acid mono-2-ethylhexanoic acid glyceryl | 0.5 mass% |
| dimethyl distearyl ammonium hectorite | 1 mass% |
| perfume | appropriate mass% |
| purified water | residue |

Using each cosmetic of the Example 5-17, it was prevented to come off and kept long.

## Claims

1. Cosmetics containing polyurethane particles wherein;
each surface of the bodies of the polyurethane particles is covered with hydrophilic fine silica powders,
the body of the polyurethane particle is obtained by three-dimensionally polymerizing an isocyanate-terminated urethane prepolymer with trifunctional or more functional amines, and the isocyanate-terminated urethane prepolymer is obtained by reacting polyisocyanates and polyols including a poly(tetramethylene ether)glycol.

2. The cosmetics according to the claim 1, wherein;
each surface of the bodies of the polyurethane particles is covered with hydrophilic fine silica powders which are implanted on the surface.

3. The cosmetics according to the claim 1, wherein;
a number average molecular weight of poly(tetramethylene ether)glycol is 650-3000.

4. The cosmetics according to the claim 1, wherein;
an isophorone diisocyanate is used as the polyisocyanates and the poly(tetramethylene ether)glycol is used as the polyols.

5. The cosmetics according to the claim 1, wherein;
a 3,3'-diaminodipropylamine is used as trifunctional amines.

6. The cosmetics according to the claim 1, wherein;
a particle size of the polyurethane particle is 20 or more times larger than a particle size of the hydrophilic fine silica powder.

7. The cosmetics according to the claim 1, wherein;
an average particle size of the polyurethane particles is 1-50µm.

8. The cosmetics according to the claim 1, wherein;
the polyurethane particles are containing 0.1-30 parts by mass in the cosmetics.

9. The cosmetics according to the claim 1, wherein;
the form of the cosmetics is selected from the group consisting of solid, powder, gel and liquid.
